# EUROPEAN PATENT SPECIFICATION

(11) **EP 2 247 321 B1**
(45) Date of publication and mention of the grant of the patent: **13.03.2013**
(21) Application number: 09705363.1
(22) Date of filing: 05.01.2009
(51) Int. Cl.: A61L 31/10, A61L 31/16

(54) **ORDERED COATINGS FOR DRUG ELUTING STENTS AND MEDICAL DEVICES**
GEORDNETE BESCHICHTUNGEN FÜR ARZNEIMITTELELUTIERENDE STENTS UND MEDIZINISCHE VORRICHTUNGEN
REVÊTEMENTS ORDONNÉS POUR DES STENTS D'ÉLUTION DE MÉDICAMENT ET DISPOSITIFS MÉDICAUX

(30) Priority: 28.01.2008 US 21010
(43) Date of publication of application: 10.11.2010
(73) Proprietor: Medtronic Vascular Inc., Santa Rosa, CA 95403 (US)
(72) Inventor: TEDESCHI, Eugene, Santa Rosa California 95404 (US); FRANCIS, Richard, White Bear Lake Minnesota 55110 (US); BIRDSALL, Matthew, Santa Rosa California 95404 (US)
(74) Representative: Zimmermann & Partner
(86) International application number: PCT/US2009/030077
(87) International publication number: WO 2009/097165

(56) References cited:
- EP-A1- 1 466 634
- EP-A1- 1 518 570
- US-A1- 2006 093 771
- US-A1- 2007 280 991

## Description

### TECHNICAL FIELD

This invention relates generally to biomedical devices that are used for treating vascular conditions. More specifically, the invention relates to therapeutic agent eluting stents and other medical devices having layered coatings that release one or more therapeutic agents in a predetermined sequence at therapeutically optimal rates, as defined in the claims.

### BACKGROUND OF THE INVENTION

Stents are generally cylindrical-shaped devices that are radially expandable to hold open a segment of a vessel or other anatomical lumen after implantation into the body lumen.

Various types of stents are in use, including expandable and self-expanding stents. Expandable stents generally are conveyed to the area to be treated on balloon catheters or other expandable devices. For insertion into the body, the stent is positioned in a compressed configuration on the delivery device. For example, the stent may be crimped onto a balloon that is folded or otherwise wrapped about the distal portion of a catheter body that is part of the delivery device. After the stent is positioned across the lesion, it is expanded by the delivery device, causing the diameter of the stent to expand. For a self-expanding stent, commonly a sheath is retracted, allowing the stent to expand.

Stents are used in conjunction with balloon catheters in a variety of medical therapeutic applications, including intravascular angioplasty to treat a lesion such as plaque or thrombus. For example, a balloon catheter device is inflated during percutaneous transluminal coronary angioplasty (PTCA) to dilate a stenotic blood vessel. When inflated, the pressurized balloon exerts a compressive force on the lesion, thereby increasing the inner diameter of the affected vessel. The increased interior vessel diameter facilitates improved blood flow. Soon after the procedure, however, a significant proportion of treated vessels restenose.

To reduce restenosis, stents, constructed of metals or polymers, are implanted within the vessel to maintain lumen size. The stent is sufficiently longitudinally flexible so that it can be transported through the cardiovascular system. In addition, the stent requires sufficient radial strength to enable it to act as a scaffold and support the lumen wall in a circular, open configuration

Stent insertion and expansion may cause undesirable reactions such as inflammation resulting from a foreign body reaction, infection, thrombosis, and proliferation of cell growth that occludes the blood vessel. Stents capable of delivering one or more therapeutic agents have been used to treat the damaged vessel and reduce the incidence of deleterious conditions including thrombosis and restenosis.

Polymer coatings applied to the surface of the stents have been used to deliver drugs or other therapeutic agents at the placement site of the stent. The coating may comprise biodegradable or biostable polymers singly, or in various combinations to give the coating unique properties such as controlled rates of degradation, or a biostable mesh with a biodegradable or bioerodable portions that control elution of the therapeutic agent.

Depending on the medical condition being treated, it is sometimes desirable to deliver more than one therapeutic agent from the surface of the stent or other medical device. For example, an anti-inflammatory agent may be delivered for a short period of time immediately after the device is placed at the treatment site, followed by an antiproliferative agent that is delivered for several weeks or months following implantation of the device. Alternatively, one therapeutic agent may be released, but at two or more different rates at different times following implantation. It would therefore be desirable, to provide an implantable therapeutic agent eluting stent or other medical implant having a layered polymeric coating capable of releasing one or more therapeutic agents in sequence and at therapeutically efficacious rates. Such a stent or medical device would overcome many of the limitations and disadvantages inherent in the devices described above.

US 2007/0280991 describes coatings for implantable devices for controlled release of a hydrophilic drug and a hydrophobic drug. US 2006/0093771 describes a polymer coating for medical devices.

### SUMMARY OF THE INVENTION

One aspect of the present invention provides a system for treating a vascular condition comprising a catheter and a therapeutic agent-carrying stent disposed on the catheter. The stent includes a layered coating disposed on the surface of the stent, and at least one therapeutic agent contained within the coating. The layers of the coating provide an ordered release of the therapeutic agent in sequenced phases that are optimal for treatment of the patient.

The layered coating further comprises a first layer having a first polymer soluble in a first solvent and a second layer having a second polymer soluble in a second solvent wherein the polymer of the first layer is insoluble in the second solvent, wherein the polymer of the first layer comprises an aqueous soluble polymer selected from the group consisting of polylactic acid, polyglycolic acid and their copolymers, polyethylene glycol, polyacetates, poloxamers, poloxamines, polyamide, starch, sugar, dextran, cellulose, alginate, hyaluronic acid and combinations thereof; and wherein the polymer of the second layer comprises a nonaqueous soluble polymer selected from the group consisting of polyanhydrides, polyurethanes, polycaprolactones, poly(ortho esters), polyethylene, polypropylene, polymethyl methacrylate, polyesters, polyamides, ethylene vinyl alcohol copolymer, polytetrafluoroethylene (PTFE), polyvinyl alcohol, and combinations thereof.

Another aspect of the invention provides a stent having a layered coating on at least a portion of the surface of the stent, and at least one therapeutic agent within the coating. The layers of the coating provide an ordered release of the therapeutic agent in sequenced phases that are optimal for treatment.

The layered coating further comprises a first layer having a first polymer soluble in a first solvent and a second layer having a second polymer soluble in a second solvent wherein the polymer of the first layer is insoluble in the second solvent,
wherein the polymer of the first layer comprises an aqueous soluble polymer selected from the group consisting of polylactic acid, polyglycolic acid and their copolymers, polyethylene glycol, polyacetates, poloxamers, poloxamines, polyamide, starch, sugar, dextran, cellulose, alginate, hyaluronic acid and combinations thereof; and wherein the polymer of the second layer comprises a nonaqueous soluble polymer selected from the group consisting of polyanhydrides, polyurethanes, polycaprolactones, poly(ortho esters), polyethylene, polypropylene, polymethyl methacrylate, polyesters, polyamides, ethylene vinyl alcohol copolymer, polytetrafluoroethylene (PTFE), polyvinyl alcohol, and combinations thereof.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims. The drawings are not to scale. The foregoing aspects and other attendant advantages of the present invention will become more readily appreciated by the detailed description taken in conjunction with the accompanying drawings.

### BRIEF DESCRIPTION OF THE DRAWINGS

FIG. 1 is a schematic illustration of a system for treating a vascular condition comprising a therapeutic agent carrying stent coupled to a catheter, in accordance with one embodiment of the present invention;

FIG. 2 is a schematic illustration of a layered coating comprising two layers including therapeutic agent(s) on the surface of a stent or other medical device in accordance with the present invention;

FIG. 3 is a schematic illustration of a layered coating comprising two therapeutic agent delivery layers separated by a tie layer on a stent or other medical device in accordance with the present invention;

FIG. 4 is a schematic illustration of release of a therapeutic agent from a layered coating on a stent or other medical device in which the release of one therapeutic agent is activated by degradation of the outer layer of the coating; and

FIG. 5 is a flow diagram of a method for treating a vascular condition using a stent with a layered coating, in accordance with the present invention.

### DETAILED DESCRIPTION

Throughout this specification, like numbers refer to like structures.

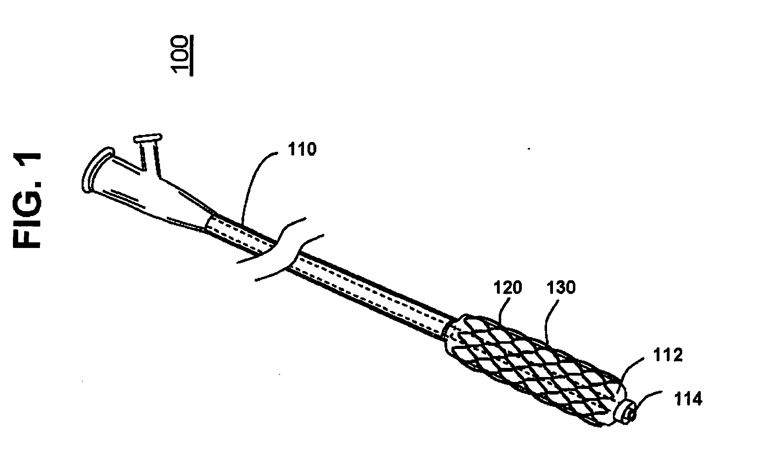

The present invention is directed to a system for treating abnormalities of the cardiovascular system comprising a catheter and a therapeutic agent-carrying stent disposed on the catheter. A layered coating disposed on the surface of the stent releases one or more therapeutic agents in an ordered sequence. In an exemplary embodiment of the invention, FIG. 1 shows an illustration of a system 100 comprising therapeutic agent carrying stent 120 coupled to catheter 110. Catheter 110 includes a balloon 112 that expands and deploys therapeutic agent carrying stent 120 within a vessel of the body. After positioning therapeutic agent carrying stent 120 within the vessel with the assistance of a guide wire traversing through guide wire lumen 114 inside catheter 110, balloon 112 is inflated by pressurizing a fluid such as a contrast fluid or saline solution that fills a lumen inside catheter 110 and balloon 112. Therapeutic agent carrying stent 120 is expanded until a desired diameter is reached; then the contrast fluid is depressurized or pumped out, separating balloon 112 from therapeutic agent carrying stent 120 and leaving the therapeutic agent carrying stent 120 deployed in the vessel of the body. Alternately, catheter 110 may include a sheath that retracts to allow expansion of a self-expanding embodiment of therapeutic agent carrying stent 120. Therapeutic agent carrying stent 120 includes a stent framework 130 forming interior and exterior surfaces of the stent. A layered coating is disposed on the surface of at least a portion of stent framework 130.

In one embodiment of the invention, the stent framework comprises one or more of a variety of biocompatible metals such as stainless steel, titanium, magnesium, aluminum, chromium, cobalt, nickel, gold, iron, iridium, chromium/titanium alloys, chromium/nickel alloys, chromium/cobalt alloys, such as MP35N and L605, cobalt/titanium alloys, nickel/titanium alloys, such as nitinol, platinum, and platinum-tungsten alloys. The metal composition gives the stent framework the mechanical strength to support the lumen wall of the vessel, sufficient longitudinal flexibility so that it can be transported through the cardiovascular system, and provides a metallic substrate for the oxidation and reduction reactions that produce a porous coating.

In another embodiment of the invention, stent framework 130 comprises one or more biocompatible polymeric materials. Polymeric stent framework 130 may be biodegradable, biostable, or comprise a mixture of polymeric materials that are both biostable and biodegradable. Biodegradable polymers appropriate for the stent framework of the invention include polylactic acid, polyglycolic acid, and their copolymers, caproic acid, polyethylene glycol, polyanhydrides, polyacetates, polycaprolactones, poly(orthoesters), polyamides, polyurethanes and other suitable polymers. Biostable polymers appropriate for the stents of the invention include polyethylene, polypropylene, polymethyl methacrylate, polyesters, polyamides, polyurethanes, polytetrafluoroethylene (PTFE), polyvinyl alcohol, other biostable polymers, and combinations thereof. These polymers may be used alone or in various combinations to give the stent framework desirable properties such as flexibility, coating durability and controlled rates of degradation.

The stent framework is formed by shaping a metallic wire or polymeric filament, or by laser cutting the stent from a metallic or polymeric sheet, compression molding of polymer pellets to form a filament, or any other appropriate method. If needed, the surface of the stent framework is cleaned by washing with surfactants and/or organic solvents to remove oils, mechanical polishing, electropolishing, etching with acid or base, or any other effective means to expose a clean, uniform surface that is ready for applying a coating.

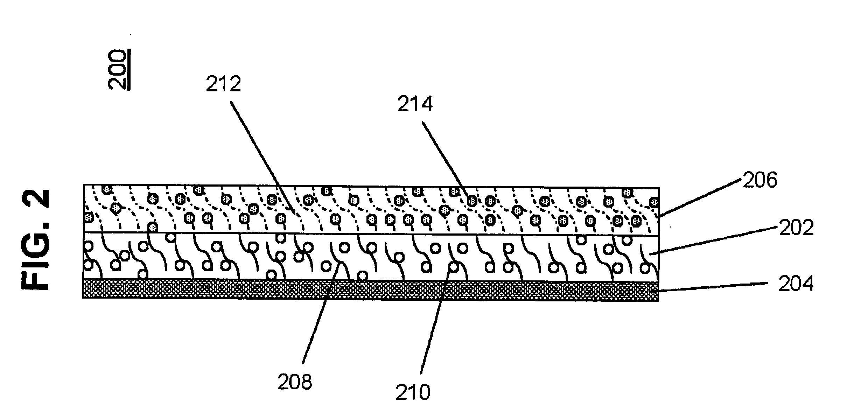

FIG. 2 is an illustration of a layered stent coating 200 comprising two therapeutic agent delivery layers: a first therapeutic agent delivery layer 202, disposed on surface 204 of a stent framework or other medical device, and a second therapeutic agent delivery layer 206 overlaying layer 202. In one embodiment, layer 206 covers a portion of layer 202, for example the portion of layer 202 disposed on the exterior portion of the stent. In a second embodiment, layer 206 completely surrounds and covers layer 202. Each coating layer 202 and 206 includes a polymer matrix 208 and 212, respectively, comprising one or more polymers that provide a delivery system appropriate for the therapeutic agent to be delivered from each layer 202 and 206. In addition, the polymers that comprise layer 202 adhere tightly to the stent surface, and the polymers that comprise layer 206 adhere to the surface of layer 202.

Polymers 208, comprising layer 202, are insoluble in one or more solvents in which polymers 212 are soluble. Similarly, the polymers 212, comprising layer 206, are insoluble in solvents capable of dissolving polymers 208. In one embodiment, the polymers comprising one layer, for example, layer 206, are water soluble, and the polymers comprising the second layer, in this example layer 202, are insoluble in water, but are soluble in organic solvents. Aqueous soluble polymers appropriate for these coatings include polylactic acid, polyglycolic acid, and their copolymers, polyethylene glycol, polyacetates, poloxamers, poloxamines, polyamides, starch sugar, dextran, cellulose, alginate, hyaluronic acid, other aqueous soluble polymers. These polymers may be used alone or in combination to formulate an aqueous soluble polymer layer that will deliver the therapeutic agent at an optimal rate, and over a suitable period of time. Suitable organic soluble polymers that are minimally soluble in water include polyanhydrides, polyurethanes, polycaprolactones, poly(ortho esters), polyethylene, polypropylene, polymethyl methacrylate, polyesters, polyamides, ethylene vinyl alcohol copolymer, polytetrafluoroethylene (PTFE), polyvinyl alcohol, and other polymers. These polymers may be used alone or in various combinations to formulate an organic soluble polymer layer that will deliver one or more therapeutic agents at an optimal rate.

In one embodiment, one or more polymers 208 are selected that will deliver therapeutic agent 210 from coating layer 202 at a therapeutically effective dose and time course. Polymers 208 are dissolved or suspended in an appropriate solvent or solvent mixture, and applied to the surface 204 of a stent or other medical device to form coating layer 202. In some embodiments, polymeric layer 202 is applied to stent surface 204 by spraying or dipping so that a uniform polymeric layer is formed on the surface of stent framework 130. In one embodiment, a liquid polymeric formulation is sprayed on the outer surface of stent framework 130, and forms layer 202 having a uniform thickness. If needed, polymeric layer 202 is then cured by exposure to ultraviolet light, heat, gamma irradiation or any other appropriate means so that chemical cross links form among the polymer strands. Next, the coating is dried by exposure to vacuum, heat, and/or air to remove excess solvent. Finally, therapeutic agent 210 is infused into polymeric layer 202. The application process for therapeutic agent 210 may include elevated pressure or vacuum to infuse the formulation containing therapeutic agent 210 into the porous polymeric layer 202. Alternatively, therapeutic agent 210 may be blended into a formulation containing the polymeric constituents of the coating layer and applied directly to the surface 204 of stent framework 130 by any means known in the art such as, for example, by spraying or dipping stent framework 130.

After coating layer 202 is applied to stent surface 204, layer 206 is overlaid on the surface of layer 202. The polymers that form polymer mesh 212 are selected to deliver therapeutic agent 214 in a therapeutically effective amount and over an optimal time course, and also are soluble in a solvent or solvent system that does not dissolve coating layer 202. Coating layer 206 is applied to the surface of coating layer 202 by spraying or dipping, then cured and dried as needed. Finally, therapeutic agent 214 is infused into layer 206, and the stent coating is dried if needed.

Alternatively, in one embodiment, layers 202 and 206 are applied to stent surface 204 simultaneously by coextrusion of polymer matrices 208 and 212. Examples of suitable polymers for coextrusion include polyethylene, polypropylene, polyether block amide, polyethylene terephthalate, polyetherurethane, polyesterurethane, other polyurethanes, natural rubber, rubber latex, synthetic rubbers, polyester-polyether copolymers, polycarbonates, polyanhydrides, polycaprolactones, poly(ortho esters), polymethyl methacrylate, polyesters, ethylene vinyl alcohol copolymer, polytetrafluoroethylene (PTFE), polyvinyl alcohol, and other polymers. These polymers may be used alone or in combination to provide polymeric matrices 208 and 212 having suitable characteristics.

In one embodiment of the invention, therapeutic agent molecules 210 and 214 are contained within coating layers 202 and 206, respectively. Therapeutic agents 210 and 214 may be the same or different substances. Various therapeutic agents, such as anticoagulants, antiinflammatories, fibrinolytics, antiproliferatives, antibiotics, therapeutic proteins or peptides, recombinant DNA products, or other bioactive agents, diagnostic agents, radioactive isotopes, or radiopaque substances may be used depending on the anticipated needs of the targeted patient population. The formulation containing the therapeutic agent may additionally contain excipients including solvents or other solubilizers, stabilizers, suspending agents, antioxidants, and preservatives, as needed to deliver an effective dose of the therapeutic agent to the treatment site.

Therapeutic agent molecules 210 and 214 are held within coating layers 202 and 206 by entrapment within the polymer mesh of the coating, or by chemical means such as hydrogen bonding, or hydrophobic interactions depending on the polarity and solubility of therapeutic agent molecules 210 and 214.

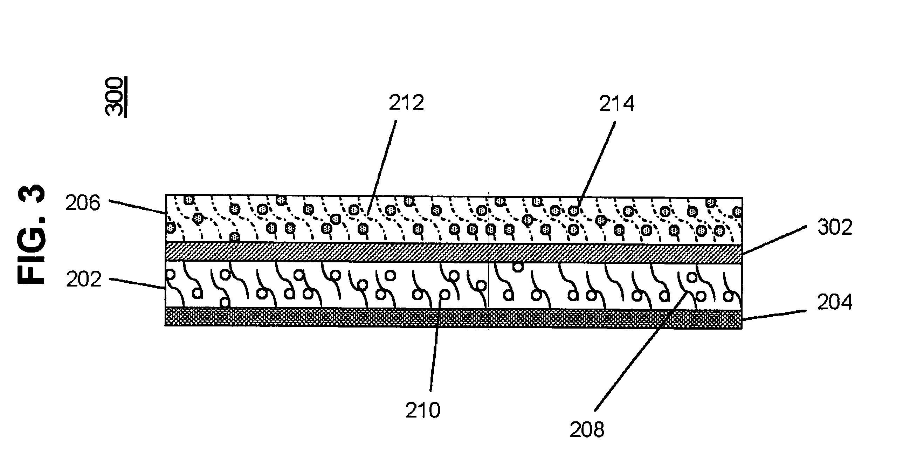

In one embodiment, a first therapeutic agent delivery layer 202 and a second therapeutic agent delivery layer 206 are separated by tie layer 302, as shown in FIG. 3. In one embodiment, tie layer 302 comprises one or more polymers that form an elastomeric film and provide tie layer 302 with sufficient flexibility to prevent layers 202 and 206 from delaminating during expansion and contraction of stent framework 130. In one embodiment, tie layer 302 comprises suitably elastic polymers such as polyurethane-latex, polyurethane, or polybutadiene, alone or with other polymers. Other polymers with appropriate elastomeric properties include polyether block amide, polyethylene terephthalate, polyetherurethane, polyesterurethane, other polyurethanes, natural rubber, rubber latex, synthetic rubbers, polyester-polyether copolymers, polycarbonates, polyanhydrides, polycaprolactones, poly(ortho esters), polyethylene, polymethyl methacrylate, polyesters, ethylene vinyl alcohol copolymer, polyvinyl alcohol, and other elastomeric polymers. These polymers may be used alone or in combination to provide a tie layer having suitable elastomeric characteristics.

In one embodiment, it is desirable to select the polymers for layer 206 that provide a lubricious outer coating layer 206 to facilitate delivery and placement of the stent. However, such polymers generally do not adhere well to the surface of layer 202, resulting in delamination of coating layers 202 and 206. It is therefore desirable to use a tie layer that binds to both polymeric matrices 208 and 212. In one embodiment, polymer matrix 208 comprises poly-butyl-methacrylate polymers, and delivers the antiproliferative agent, zotarolimus (therapeutic agent 202). Polymer matrix 212 comprises polyethylene oxide and provides a lubricious outer layer of the coating and delivers the anti-inflamatory agent, paclitaxel (therapeutic agent 214). Because poly-butyl-methacrylate and polyethylene oxide do not adhere well to each other, a tie layer comprising a polymeric matrix of water soluble polyurethane-latex is used to bind therapeutic agent delivery layers 202 and 206 tightly together and provide sufficient elasticity to prevent cracking and delamination of the layered coating during expansion and contraction of stent framework 130.

In one embodiment, tie layer 302 forms a barrier between layers 202 and 206. In this embodiment both therapeutic agents 210 and 214 are at least partially soluble in various solvent systems. Therefore a first coating layer 202 is applied to stent surface 204, and therapeutic agent 210 is infused into coating layer 202. If second layer 206 of the coating were next applied and therapeutic agent 214 were infused in a solvent in which therapeutic agent 210 is at least partially soluble, therapeutic agent 210 would leach out of layer 202 during application of therapeutic agent 214. Therefore, in one embodiment, a biodegradable tie layer that is impermeable to the solvent to be used for application of therapeutic agent 214 is overlaid on the surface of layer 202. Some biodegradable polymers that have increased solvent resistance include_polyesters, polyesteramides, polyesterurethanes, thermoplastic starch, and other natural polymers.

In one exemplary embodiment, therapeutic agent 210 is zotarolimus which is soluble in polar organic solvents such as methanol, acetone and chloroform, but only slightly soluble in water. In this example, tie layer 302 comprises polyesters or polyesterurethanes that are soluble only in nonpolar organic solvents such as hexane. In this example, therapeutic agent 210 (zotarolimus) will remain within layer 202 during application of tie layer 302. Polymer matrix 212 comprises polyethylene oxide, a polymer of varying molecular weight that is soluble in water/isopropyl alcohol mixtures. Polymer matrix 212 may be applied over tie layer 302 without affecting the zotarolimus concentration in layer 202.

In one embodiment, after delivery to the treatment site, therapeutic agent molecules 214 near the surface of layer 206 are rapidly released by migration of therapeutic agent molecules 214 through the coating and out from the surface of layer 206. In another embodiment coating layer 206 comprises polymers that are biodegradable under physiological conditions, and coating layer 206 begins to degrade soon after placement of the stent or other medical device at the treatment site. In this embodiment, as polymer matrix 206 degrades, therapeutic agent molecules 214 that were entrapped within coating layer 206 are released at a rate determined by the rate of breakdown of coating layer 206. In one embodiment polymers 212 are selected that have a rate of breakdown that provides a therapeutically effective amount of therapeutic agent 214 at the treatment site resulting from release of therapeutic agent 214 during degradation of coating layer 206.

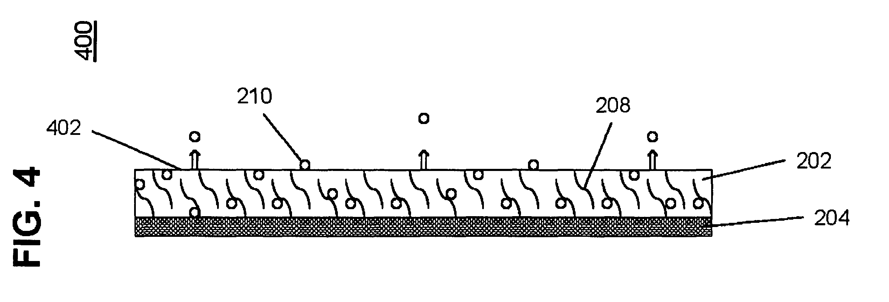

After coating layer 206 has degraded and been removed, surface 402 of layer 202 is exposed, as shown in FIG. 4. Therapeutic agent 210 is then released by diffusion through surface 402, resulting in a sequential release of therapeutic agent 214 followed by therapeutic agent 210. In one embodiment, therapeutic agent 214 is an anti-inflammatory agent such as paclitaxel, dexamethasone, hydrocortisone, salicylic acid, fluocinolone acetonide, corticosteroids and other drugs and prodrugs. Therapeutic agent 210 is an antiproliferative such as zotarolimus, sirolimus, everolimus, pimecrolimus, and other drugs having antiproliferative activityl. Polymer matrix 212 is biodegradable and comprises poly-lactide, and glycolide polymers. In this example, therapeutic agent 214, the anti-inflammatory agent, is released soon after implantation of the stent or other device, reducing the inflammatory tissue reaction in tissues surrounding the stent. Therapeutic agent 210, the antiproliferative in this example, is released days or weeks later, depending on the time needed for the poly-lactide-co-glycolide coating layer 206 to degrade and be removed thereby exposing layer 202 and initiating release of therapeutic agent 210.

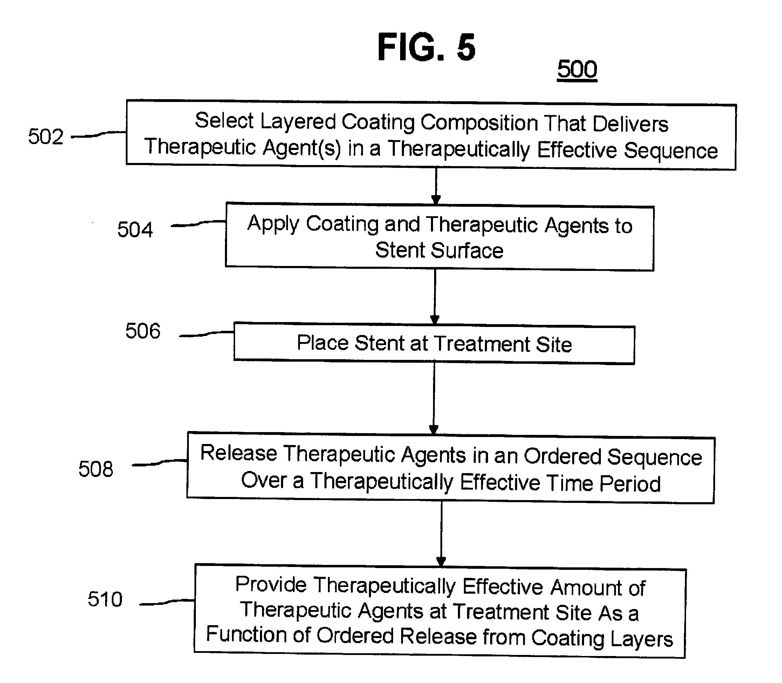

FIG. 5 is a flowchart of method 500 for treating a vascular condition using a stent having a layered therapeutic agent eluting coating, in accordance with the present invention. The method includes selecting polymers for at least two coating layers that will release therapeutic agents at the treatment site in a sequenced order and over a desired time period, as indicated in Block 502. The coating comprises a polymer matrix, one or more therapeutic agents to be delivered, and any other excipients needed to cause the coating to adhere to the surface of the stent framework, and deliver the therapeutic agent(s) to the treatment site. The polymer matrix is either biodegradable or biostable. The therapeutic agents are held within each coating layer by entrapment or chemical bonding, and are released at the treatment site by diffusion out of the coating, or as a result of biodegradation of the coating. In some embodiments, the therapeutic agent delivery layers are separated by a tie layer that prevents migration of the therapeutic agents between the layers, improves adherence of the layers to each other to form a robust coating, and provides elasticity to the coating to prevent chipping and delamination during expansion and contraction of the stent framework.

As indicated in Block 504, the coating is applied to the surface of the stent frame work. In one embodiment of the invention, the polymeric matrix comprising the first coating layer is applied as a liquid by dipping or spraying, then cured if necessary, and dried to remove excess solvent using air, vacuum, heat, or any other effective means of causing the coating layer to adhere to the stent framework. The therapeutic agent to be delivered from that layer is then infused into the coating layer. Next, the tie layer, if used is overlaid on the surface of the first coating layer, and finally the second therapeutic agent delivery layer is applied to the surface of the previous layer using methods similar to those described for the first layer. Finally the second therapeutic agent is infused into the outer layer leaving the underlying layers and the first therapeutic agent undisturbed. Alternatively, in one embodiment the layers are applied simultaneously by coextrusion of the polymer matrices comprising each coating layer.

Next, as indicated in Block 506, the coated, therapeutic agent eluting stent is mounted on a catheter and delivered to the treatment site. At the treatment site, the stent is positioned across the lesion to be treated and expanded. The catheter is then withdrawn from the body.

In the physiological environment, the therapeutic agents are released from the layered coating in an ordered sequence and over a therapeutically effective time period as indicated in Block 508. In one embodiment, therapeutic agent molecules in the outer layer of the coating migrate out of the coating and deliver a therapeutically effective amount of the therapeutic agent at the treatment site immediately after placement of the stent.

In another embodiment the outer layer of the coating biodegrades, and releases of the therapeutic agent within the outer layer beginning immediately after placement of the stent, and at a rate controlled by the degradation of the coating. Next, the inner layer of the coating is exposed, and the therapeutic agent within that layer is released over a time period determined by the nature of the inner layer of the coating. Thus, release of the second therapeutic agent is delayed for a period time following placement of the stent, and is initiated only after most of the first therapeutic agent is released. This embodiment provides an ordered sequence of release of two different therapeutic agents from the layered coating as indicated in Block 510.

## Claims

1. A stent (120) having a layered coating (200) disposed on at least a portion of the surface (204) of the stent (120) and at least one therapeutic agent (210, 214) within the coating, wherein the layers of the coating provide an ordered release of the therapeutic agent (210, 214) in a sequence optimal for treatment, wherein the layered coating (200) further comprises a first layer (202) having a first polymer (208) soluble in a first solvent and a second layer (206) having a second polymer (212) soluble in a second solvent wherein the polymer (208) of the first layer (202) is insoluble in the second solvent
wherein the polymer of the first layer (202) comprises an aqueous soluble polymer selected from the group consisting of polylactic acid, polyglycolic acid and their copolymers, polyethylene glycol, polyacetates, poloxamers, poloxamines, polyamide, starch, sugar, dextran, cellulose, alginate, hyaluronic acid, and combinations thereof; and
wherein the polymer of the second layer (206) comprises a nonaqueous soluble polymer selected from the group consisting of polyanhydrides, polyurethanes, polycaprolactones, poly(ortho esters), polyethylene, polypropylene, polymethyl methacrylate, polyesters, polyamides, ethylene vinyl alcohol copolymer, polytetrafluoroethylene (PTFE), polyvinyl alcohol, and combinations thereof.

2. The stent (120) of claim 1 wherein the layered coating (200) comprises a first polymeric therapeutic agent release layer (202) and a second polymeric therapeutic agent release layer (206) separated by a tie layer (302).

3. The stent (120) of claim 2 wherein the tie layer (302) comprises at least one elastic polymer.

4. The stent (120) of claim 3 wherein the at least one elastic polymer is selected from the group consisting of polyurethane latex, polyurethane, polybutadiene, polyesters, polyesteramides, thermoplastic starch, and other natural polymers.

5. The stent (120) of claim 1 wherein the at least one therapeutic agent (210, 214) is selected from the group consisting of anticoagulants, antiinflammatories, fibrinolytics, antiproliferatives, antibiotics, therapeutic proteins, recombinant DNA products, bioactive agents, diagnostic agents, radioactive isotopes and radiopaque substances.

6. The stent (120) of claim 5 further comprising the first polymeric layer (202, 206) having a first therapeutic agent (210, 214) and the second polymeric layer (202, 206) disposed on the first polymeric layer (202, 206) having a second therapeutic agent (210, 214).

7. The stent (120) of claim 6 wherein the first therapeutic agent (210, 214) comprises an antiproliferative agent and the second therapeutic agent (210, 214) comprises an anti-inflammatory agent.

8. The stent (120) of claim 5 wherein the therapeutic agent (210, 214) is selected from the group consisting of zotarolimus, everolimus, sirolimus, pimecrolimus, dexamethasone, hydrocortisone, salicylic acid, fluocinolone acetonide, corticosteroids, and combinations thereof.

9. A system (100) for treating a vascular condition comprising:
a catheter (110); and
a stent (120) according to any of preceding claims disposed on the catheter (110).

## Patentansprüche

1. Stent (120) mit einer mehrlagigen Beschichtung (200), die auf mindestens einem Teil der Oberfläche (204) des Stents (120) angeordnet ist, und mit mindestens einem therapeutischen Mittel (210, 214) in der Beschichtung, wobei die Schichten der Beschichtung eine geordnete Freisetzung des therapeutischen Mittels (210, 214) in einer zur Behandlung optimalen Abfolge bereitstellen, wobei die mehrlagige Beschichtung (200) ferner eine erste Schicht (202) mit einem ersten Polymer (208), das in einem ersten Lösungsmittel löslich ist, und eine zweite Schicht (206) mit einem zweiten Polymer (212) umfasst, das in einem zweiten Lösungsmittel löslich ist, wobei das Polymer (208) der ersten Schicht (202) in dem zweiten Lösungsmittel unlöslich ist,
wobei das Polymer der ersten Schicht (202) ein wasserlösliches Polymer ausgewählt aus der Gruppe bestehend aus Polymilchsäure, Polyglykolsäure und deren Copolymeren, Polyethylenglykol, Polyacetaten, Poloxameren, Poloxaminen, Polyamid, Stärke, Zucker, Dextran, Cellulose, Alginat, Hyaluronsäure und Kombinationen davon umfasst, und
wobei das Polymer der zweiten Schicht (206) ein nicht wasserlösliches Polymer ausgewählt aus der Gruppe bestehend aus Polyanhydriden, Polyurethanen, Polycaprolactonen, Poly(orthoestern), Polyethylen, Polypropylen, Polymethylmethacrylat, Polyestern, Polyamiden, Ethylen/Vinylalkohol-Copolymer, Polytetrafluorethylen (PTFE), Polyvinylalkohol und Kombinationen davon umfasst.

2. Stent (120) nach Anspruch 1, wobei die mehrlagige Beschichtung (200) eine erste therapeutisches Mittel freisetzende Polymerschicht (202) und eine zweite therapeutisches Mittel freisetzende Polymerschicht (206) umfasst, die durch einen Haftvermittler (302) getrennt sind.

3. Stent (120) nach Anspruch 2, wobei der Haftvermittler (302) mindestens ein elastisches Polymer umfasst.

4. Stent (120) nach Anspruch 3, wobei das mindestens eine elastische Polymer ausgewählt ist aus der Gruppe bestehend aus Polyurethanlatex, Polyurethan, Polybutadien, Polyestern, Polyesteramiden, thermoplastischer Stärke und anderen natürlichen Polymeren.

5. Stent (120) nach Anspruch 1, wobei das mindestens eine therapeutische Mittel (210, 214) ausgewählt ist aus der Gruppe bestehend aus Antikoagulantien, Antiphlogistika, Fibrinolytika, Antiproliferativa, Antibiotika, therapeutischen Proteinen, rekombinanten DNA-Produkten, bioaktiven Mitteln, Diagnostika, radioaktiven Isotopen und röntgendichten Substanzen.

6. Stent (120) nach Anspruch 5, der ferner die erste Polymerschicht (202, 206) mit einem ersten therapeutischen Mittel (210, 214) und die auf der ersten polymeren Schicht (202, 206) angeordnete zweite Polymerschicht (202, 206) mit einem zweiten therapeutischen Mittel (210, 214) umfasst.

7. Stent (120) nach Anspruch 6, wobei das erste therapeutische Mittel (210, 214) ein Antiproliferativ umfasst und das zweite therapeutische Mittel (210, 214) ein Antiphlogistikum umfasst.

8. Stent (120) nach Anspruch 5, wobei das therapeutische Mittel (210, 214) ausgewählt ist aus der Gruppe bestehend aus Zotarolimus, Everolimus, Sirolimus, Pimecrolimus, Dexamethason, Hydrocortison, Salicylsäure, Fluocinolonacetonid, Corticosteroiden und Kombinationen davon.

9. System (100) zur Behandlung einer Gefäßerkrankung, umfassend:
einen Katheter (110) und
einen Stent (120) gemäß einem der vorhergehenden Ansprüche, der auf dem Katheter (110) angeordnet ist.

## Revendications

1. Endoprothèse vasculaire (120) comportant un revêtement en couches (200) disposé sur au moins une partie de la surface (204) de l'endoprothèse vasculaire (120) et au moins un agent thérapeutique (210, 214) au sein du revêtement, dans laquelle les couches du revêtement permettent une libération ordonnée de l'agent thérapeutique (210, 214) selon une séquence optimale pour le traitement, le revêtement en couches (200) comprenant en outre une première couche (202) comportant un premier polymère (208) soluble dans un premier solvant et une deuxième couche (206) comportant un deuxième polymère (212) soluble dans un deuxième solvant et le polymère (208) de la première couche (202) étant insoluble dans le deuxième solvant
dans laquelle le polymère de la première couche (202) comprend un polymère hydrosoluble choisi dans l'ensemble constitué d'un polyacide lactique, d'un polyacide glycolique et de leurs copolymères, d'un polyéthylène glycol, de polyacétates, de poloxamères, de poloxamines, d'un polyamide, d'amidon, de sucre, de dextrane, de cellulose, l'alginate, d'acide hyaluronique et de leurs combinaisons ; et
dans laquelle le polymère de la deuxième couche (206) comprend un polymère non hydrosoluble choisi dans l'ensemble constitué de polyanhydrides, de polyuréthanes, de polycaprolactones, de poly(ortho esters), de polyéthylène, de polypropylène, de poly(méthacrylate de méthyle), de polyesters, de polyamides, de copolymère d'éthylène et d'alcool vinylique, de polytétrafluoroéthylène (PTFE), de poly(alcool vinylique) et de leurs combinaisons.

2. Endoprothèse vasculaire (120) selon la revendication 1, dans laquelle le revêtement en couches (200) comprend une première couche (202) d'agent thérapeutique polymère et une deuxième couche (206) d'agent thérapeutique polymère, séparée par une couche (302) de liaison.

3. Endoprothèse vasculaire (120) selon la revendication 2, dans laquelle la couche (302) de liaison comprend au moins un polymère élastique.

4. Endoprothèse vasculaire (120) selon la revendication 3, dans laquelle le polymère élastique, au nombre d'au moins un, est choisi dans l'ensemble constitué de latex de polyuréthane, de polyuréthane, de polybutadiène, de polyesters, de polyesteramides, d'amidon thermoplastique et d'autres polymères naturels.

5. Endoprothèse vasculaire (120) selon la revendication 1, dans laquelle l'agent thérapeutique (210, 214), au nombre d'au moins un, est choisi dans l'ensemble constitué d'anticoagulants, d'anti-inflammatoires, de fibrinolytiques, d'antiprolifératifs, d'antibiotiques, de protéines thérapeutiques, de produits de l'ADN recombiné, d'agents bioactifs, d'agents de diagnostic, d'isotopes radioactifs et de substances radio-opaques.

6. Endoprothèse vasculaire (120) selon la revendication 5, comprenant en outre la première couche polymère (202, 206) comportant un premier agent thérapeutique (210, 214) et la deuxième couche polymère (202, 206) disposée sur la première couche polymère (202, 206) comportant un deuxième agent thérapeutique (210, 214).

7. Endoprothèse vasculaire (120) selon la revendication 6, dans laquelle le premier agent thérapeutique (210, 214) comprend un agent antiprolifératif et où le deuxième agent thérapeutique (210, 214) comprend un agent anti-inflammatoire.

8. Endoprothèse vasculaire (120) selon la revendication 5, dans laquelle l'agent thérapeutique (210, 214) est choisi dans l'ensemble constitué de zotarolimus, d'everolimus, de sirolimus, de pimécrolimus, de dexaméthasone, d'hydrocortisone, d'acide salicylique, de fluocinolone acétonide, de corticostéroïdes et de leurs combinaisons.

9. Système (100) de traitement d'un état vasculaire comprenant :
un cathéter (110) ; et
une endoprothèse vasculaire (120) selon l'une quelconque des revendications précédentes, disposée sur le cathéter (110).
